Europäisches Patentamt

European Patent Office

Office européen des brevets

(11) Publication number: **0 413 533 A1**

## EUROPEAN PATENT APPLICATION

(21) Application number: 90308863.1

(22) Date of filing: **10.08.90**

(51) Int. Cl.⁵: **A61K 9/50**, A61K 9/52

(30) Priority: **14.08.89 US 392770**

(43) Date of publication of application:
**20.02.91 Bulletin 91/08**

(84) Designated Contracting States:
**AT BE CH DE DK ES FR GB GR IT LI LU NL SE**

(71) Applicant: **EURAND AMERICA, INCORPORATED**
**845 Center Drive**
**Vandalia, Ohio 45377(US)**

(72) Inventor: **Powell, Thomas Clark**
**1536 Preble County Line Road**
**West Alexandria, Ohio 45381(US)**
Inventor: **Calanchi, Massimo Maria**
**Via Calatafimi No 12**
**I-20052 Monza(IT)**

(74) Representative: **Porter, Graham Ronald et al**
**c/o Wyeth Laboratories Huntercombe Lane**
**South**
**Taplow Maidenhead Berkshire SL6 0PH(GB)**

(54) Microencapsulated taste-masked water-insoluble NSAID drug materials.

(57) This disclosure is directed to preparation of individual taste-masked, high payload, microcapsules by microencapsulation of water-insoluble NSAID drug materials in the substantial absence of microcapsule agglomerates. These taste-masked microcapsules contain a high payload, e.g. at least 83 + wt.% of said NSAID drug material and can then be formulated into chewable tablets and liquid aqueous suspensions for medicinal use. Cellulose acetate phthalate is the micro-encapsulating polymer wall material.

Control of pH, controlled addition of a Hofmeister (lyotropic), salt, microencapsulation of the water-insoluble NSAID medicament with a liquid phase of cellulose acetate phthalate microencapsulating material and the subsequent insolubilization of said liquid encapsulating material after it is wrapped around the medicament core with dilute acid are important process parameters to achieving the proper individual microcapsules to obtain taste-masked, water insoluble NSAID drug materials, esp., naproxen and ibuprofen, by microencapsulation alone.

## MICROENCAPSULATED TASTE-MASKED WATER-INSOLUBLE NSAID DRUG MATERIALS

### BRIEF DESCRIPTION OF THE INVENTION

The present invention is directed to preparation of individual taste-masked, high payload, microcapsules by micro- encapsulation of water-insoluble NSAID drug materials in the substantial absence of microcapsule agglomerates. These taste-masked microcapsules contain a high payload, e.g., at least 83 + wt.% of said NSAID drug material and can then be formulated into chewable tablets and liquid aqueous suspensions for medicinal use. Cellulose acetate phthalate is the microencapsulating polymer wall material.

Control of pH, controlled addition of a Hofmeister (lyo-tropic) salt, microencapsulation of the water-insoluble NSAID medicament with a liquid phase of cellulose acetate phthalaTe microencaPsulating material and the subsequent insolubilization of said liquid encapsulating material after it is wrapped around the medicament core with dilute acid are important process parameters to achieving the proper individual microcapsules to obtain taste-masked, water-insoluble NSAID drug materials, e.g., naproxen and ibuprofen, by microencapsulation alone.

### BACKGROUND OF THE INVENTION AND PRIOR ART

Non-steroidal anti-inflammatory drugs (NSAID) having analgesic and anti-inflammatory properties have been widely administered orally in the treatment of mild to severe pain, particularly for rheumatoid arthritis and osteoarthritis patients. Tolerance or addiction to these drugs is not generally a problem with their continuous use in the treatment of pain or in the treatment of acute or chronic inflammatory states. However, these drugs generally have a higher potential for adverse side effects at the upper concentrations (limits) of their effective dose ranges. Therefore, it is important that such non-steroidal anti-inflammatory drugs be accurately measured and administered orally.

These non-steroidal anti-inflammatory drugs, e.g., ibuprofen and naproxen, have been widely prescribed by physicians. These drugs are in general tolerated well by most patients and provide an effective means for control of pain and inflammatory processes, particularly for the rheumatoid arthritis and osteoarthritis patients. However, these non-steroidal anti-inflammatory drugs have severe bitter taste and aftertaste, and have an adverse mouth feel when taken orally.

Therefore, in order to make wider use of them while substantially eliminating the bitter taste, aftertaste and adverse mouth feel and make these drugs more pleasant upon taking them orally, there has long been desired a way to insure delivery of these drugs in their desired concentrations while avoiding their extremely bitter taste, lingering aftertaste and adverse mouth feel effects referred to above connected with their ingestion orally thereby encouraging patient compliance.

Various ways and delivery systems have been attempted in the prior art to accomplish these and other objectives.

U.S. patent No. 4,755,532, issued to A. Sunshine et al, is directed to analgesic and anti-inflammatory compositions comprising diphenhydramine and analgesically and anti-inflammatorily effective amounts of sulindac, diclofenac, fenclofenac, alclofenac, ibufenac, isoxepac, furofenac, tiopinac, zidometacin, acemetacin, fentiazac, clidanac or oxipinac, or a pharmaceutically acceptable salt thereof. The Sunshine et al patent apparently relies upon sweetening and flavoring agents to counter the very bitter taste and lingering aftertaste of ibuprofen and naproxen when these two drugs are incorporated into the Sunshine et al compositions and they are administered orally.

U.S. Patent 4,766,012, issued to Valenti, teaches the microencapsulation of ibuprofen and naproxen. The micro encapsulation method employed by Valenti dissolves a coating agent in water by salification to form an aqueous solution, dispersing the medicament particles first in water, then in the solution of salified coating agent to form a suspension, and adding an acidifying agent thereof to precipitate the coating agent onto the particles of medicament and recovering the microcapsules thus formed.

U.S. Patent 4,460,563 issued to Massimo Calanchi, discloses that ibuprofen can be microencapsulated with hydroxy-propylmethylcellulose phthalate.

U.S. Patent 4,726,966, issued to Kawashima et al, dissolves ibuprofen with acrylic acid resin in ethanol and mixes this solution with water while stirring to deposit the coating. According to Kawashima et al, the appropriate granular ibuprofen grain size thus salvaged ranges from 10 to 2,000 micrometers. These particles are termed to be microspheres by Kawashima et al.

U.S. Patent 4,762,702 issued to Gergely et al, is directed to a pharmaceutical preparation containing ibuprofen coated with a hydrocolloid/fumaric acid coating, e.g., xanthan gum and/or maltodextrin-fumaric acid. Gergely et al rely heavily on sweetening and flavoring agents to alleviate the very bitter taste of ibuprofen. It is noted in Example 1 that 10 to 15 times the quantity of sugar as compared with ibuprofen is employed in addition to citric acid present in an amount equal to twice the quantity of ibuprofen in order to obtain what Gergely et al term a mixture which "could be made into a pleasant tasting beverage". Note Example 1 of Gergely et al.

U.S. Patent 4,571,333, issued to Hsiao et al, is directed to a controlled release tablet for once-daily oral administration of about 500 to 1200 mg of naproxen or naproxen sodium, utilizing a homogeneous matrix comprising about 4 to 9 weight percent of hydroxpropylmethycellulose having a number average molecular weight in the range of from about 80,000 to about 130,000, about 81 to 96 weight percent naproxen or naproxen sodium, 0.1 to about 2 weight percent of a pharmaceutically acceptable lubricating agent, and 0 to about 8 weight percent of other pharmaceutically acceptable excipients.

U.S. Patents 4,835,186; 4,835,187; and 4,835,188 are directed to making taste-neutral (taste-masked) ibuprofen in dry powder form. U.S. Patents 4,835,186 and U.S. 4,835,187 involve obtaining this taste-neutral ibuprofen in powder form by spray drying suspensions of colloidal silica in organic solvent solutions of ibuprofen and a cellulose material.

In U.S. Patent 4,835,186, issued to Gerald L. Reuter et al, the organic solvent is a mixture of lower alkanol, e.g., isopropanol, and ethyl acetate, and the cellulose material is cellulose acetate phthalate. This product is stated to contain about 40% to 70% by weight ibuprofen, about 15% to 50% by weight of cellulose acetate phthalate and about 5% to 40% by weight colloidal silica.

In U.S. 4,835,187, issued to Gerald L. Reuter et al, the organic solvent is a lower alkanol, e.g., isopropanol, or contains at least 50% lower alkanol, and the cellulose material is ethyl cellulose, hydroxyethyl cellulose, or hydroxypropylmethyl cellulose, alone or in admixture. The lower alkanol solvent has a colloidal silica suspended therein. This product is stated to contain about 40% to 70% by weight ibuprofen, about 15% to 50% of a cellulose material selected from the group consisting of ethyl cellulose, hydroxyethyl cellulose, hydroxypropylmethyl cellulose and admixtures thereof and about 5% to 40% by weight colloidal silica.

In U.S. 4,835,188, issued to Ying T.R. Ho et al, the taste-neutral powder form of ibuprofen is obtained by spray drying a dispersion of ibuprofen and ethyl cellulose in water having a plasticizer dissolved or suspended therein. This product is stated to contain about 63% to 77% by weight ibuprofen, about 25% to 40% by weight ethyl cellulose and about 2% to 7% weight plasticizer.

## DETAILED DESCRIPTION OF THE INVENTION

This invention enables the preparation of individual taste-masked microcapsules by microencapsulation of water insoluble NSAID drug materials in the substantial absence of microcapsule agglomerates, viz., agglomerates of individual microcapsules. These taste-masked individual microcapsules can then be formulated into chewable tablets and liquid aqueous suspensions of the appropriate dosage for medicinal use. Cellulose acetate phthalate is the microencapsulating polymer microcapsule wall material.

Control of pH, controlled addition of a Hofmeister (lyo-tropic) salt, microencapsulation of water-insoluble NSAID medicament with a liquid phase of cellulose acetate phthalate polymer material and the subsequent insolubilization of said liquid microencapsulating material, after it has wrapped around the medicament core, with dilute acid are important process parameters to achieving the proper individual microcapsules to obtain these taste-masked water-insoluble NSAID drug materials by microencapsulation alone. These individual microcapsules thus obtained are bland tasting, however, and use of flavoring agents to impart pleasant tastes to the already effectively taste-masked water-insoluble NSAID drug materials is preferred when formulating same into chewable tablets and liquid suspension oral dosage forms.

Usually the average/mean microcapsule diameter ranges from about 25 to about 600 $\mu$m. The process of this invention involves the principal steps of preparing an aqueous dispersion of the water-insoluble non-steroidal, anti-inflammatory drug (NSAID) material within an aqueous solution of cellulose acetate phthalate, said water-insoluble NSAID drug material particles having a particle size ranging from about 25 to about 500 $\mu$m at a pH of about 6 or higher wherein said solution contains from about 2 to about 8 wt.% of cellulose acetate phthalate; gradually adding with continued agitation a solution containing an inorganic Hofmeister (lyotropic) salt to form cellulose acetate phthalate in liquid phase separate from the equilibrium liquid; slowly adjusting the pH of the resulting solution by slow addi-tion of a dilute acid to a pH of about 4 or lower which renders said cellulose acetate phthalate polymer insoluble; and recover-ing the individual microscapsules thus formed.

The term inorganic Hofmeister (lyotropic) salt as used herein refers to the sulfate, citrate, tartrate, acetate chlor-ide, nitrate, bromide and iodide anion salts of sodium, potas-sium, ammonium, rubidium, cesium, magnesium, calcium, silicone, barium and lithium cations. Sodium sulfate is preferred.

While various dilute acids can be used to insolubilize the cellulose acetate phthalate polymer cell wall material, e.g., citric acid, acetic acid, fumaric acid or tartaric acid , the use of citric acid is preferred for this purpose in a concentration of about 10 to about 30 wt.% in water.

Suitable water-insoluble NSAID drug materials which can be used in accordance with this invention include, but are not necessarily limited to, the following: naproxen, ibuprofen, sulindac, dicolofenac, fenclofenac, alclofenac, ibufenac, isoxepac, furofenac, tiopinac, zidometacin, acemetacin, fentiazac, clidanac, oxipinac, zomepirac, sodium (Zomax) and pharmaceutically acceptable water-insoluble salts thereof.

The taste-masked individual microcapsules of water-insoluble NSAID drug material produced in accordance with this invention contain a high payload of the water-insoluble NSAID drug core material, e.g.,at least 83 + wt.%, based on total microcapsule weight.

This invention will be illustrated in greater detail in the examples which follow.

### EXAMPLE 1

(Preparation of Cellulose Acetate Phthalate (CAP) microencapsulated Naproxen)

In a 4-liter beaker there are added 3,280 grams of deionized water. With agitation, 45 grams of sodium bicarbonate were added to adjust the pH so as to solubilize the cellulose acetate phthalate polymer. 175 grams of particulate cellulose acetate phthalate having a particle size of about 14 mesh to 200 mesh, and more particularly about 1200 $\mu$m to about 50 $\mu$m , were added in small increments with continued agitation over 3-4 hours until all of the cellulose acetate phthalate (CAP) is added and dissolved. The resulting solution contains 5 weight percent cellulose acetate phthalate and has a pH of about 7.0 or slightly above.

200 grams of this 5% cellulose acetate phthalate aqueous bicarbonate solution and 200 grams of deionized water were added to a 1500 ml beaker. Then 100 grams of the naproxen (to be microencapsulated) having a particle size of 25 to 400 $\mu$m was added and dispersed with agitation. With continued agita-tion, there was added slowly dropwise from a separatory flask 250 ml of a 20% by weight aqueous solution of sodium sulfate. This sodium sulfate solution acts as a coacervating agent permitting the cellulose acetate phthalate polymer to come out as a liquid phase microencapsulating material wrapping the naproxen drug particles.

After the CAP liquid microcapsular walls are formed, the CAP polymer is rendered insoluble by slow addition of a citric acid solution until the total solution pH is approximately 4.0, thus rendering the CAP insoluble at that pH. Once this has been accomplished the agitation is stopped and the microcapsules are allowed to settle from solution so that the supernatant liquid may be decanted so as to rid the batch of the sodium sulfate. These individual microcapsules of naproxen are then washed with an acidified aqueous solution to further remove sodium sulfate. The washed CAP microencapsulated naproxen microcapsules are then filtered by a vacuum filter and dried for further use. These individual microcapsules contained approximately 91 wt.% naproxen for a 91% payload of the active drug.

### EXAMPLE 2

(Formulation of chewable tablets containing the CAP microencapsu-lated naproxen prepared ac-oording to Example 1)

One thousand 250 mg. active chewable naproxen tables were prepared as follows:

PROCEDURE FOR PREPARATION OF 1000 - 250 mg ACTIVE CHEWABLE NAPROXEN TABLETS

The following materials were added to a laboratory V-blender and blended for twenty minutes:
 a. 275 grams of taste-masked naproxen micro-capsules -(91.0% active),
 b. 600 grams of Mannitol USP granules,
 c. 200 grams of Avicel PH-101,
 d. 100 grams of Di Calcium Phosphate,
 e. 30 grams of Aspartame
 f. 4 grams of Tartaric Acid, and
 g. 20 grams of Peppermint Flavor.

After blending the above materials for 20 minutes, a small portion of the blend was removed into a container and 2.5 grams of magnesium stearate were added thereto. The materials were mixed by hand and returned to the V-blender and blended for 10 minutes.

Tablets were compressed from this mixture on a Stokes RB2 rotary press with 1.43 cm round, beveled edge, scored tooling. These tablets had the following physical properties:

Hardness - 14.0 Strong-Cobb units

Friability - 1.0% (20 tablets at 25 rpm - 4 min.)

Average Weight - 1230 milligrams

## EXAMPLE 3

(Preparation of a liquid suspension containing the CAP micro-encapsulated naproxen prepared according to Example 1)

The CAP microencapsulated naproxen microcapsules prepared in accordance with Example 1 above were then formulated into a liquid suspension dosage form. The preparation of the liquid suspension dosage form was accomplished as follows:

Four (4) liters of liquid suspension vehicle were prepared by the following procedure:

All of the following ingredients were dry blended:

800 grams of sucrose,

20 grams of sodium carboxymethylcellulose,

4.8 grams of xanthan gum,

4.8 grams of sodium saccharin,

46.5 milligrams of FD&C Yellow #5, and

103.5 milligrams of FD&C Yellow #6

The above dry blended materials were then dissolved in approximately 1500 ml of purified water in a 4-liter beaker with agitation. Then 4.8 grams of methyl paraben and 1.2 grams of propyl paraben were dissolved in 40 ml of USP propylene glycol and added to the above water solution. Then the following materials were added sequentially in their noted amounts:

1200 grams of light corn syrup,

8 ml of orange flavor oil,

2 grams of Tween 80, and

40 ml of 20% (W/V) aqueous solution of citric acid.

All ingredients were allowed sufficient time to mix and then there was added a sufficient quantity of purified water to bring the volume to 4 liters.

2.75 grams of the high payload (91%) CAP microencapsulated individual naproxen microcapsules prepared in accordance with Example 1 were added to 100 ml of the liquid suspension vehicle produced as indicated above to form a liquid suspension dosage form of microencapsulated naproxen.

This liquid suspension dosage form of microencapsulated naproxen was evaluated in a 10-person taste panel studied side by side along with the raw drug naproxen in the same liquid suspension vehicle and further compared with a commercial suspension being marketed under the trade designation NAPROSYN SUSPENSION (25 mg/ml). This NAPROSYN SUSPENSION 25 mg/ml contains 25 mg/ml active ingredient of naproxen in a vehicle of FD&C yellow #6, fumaric acid, imitation orange flavor, imitation pineapple flavor, magnesium aluminum silicate, methyl paraben, purified water, sodium chloride, sorbital solution, and sucrose.

The results of this 10-person taste panel were that the CAP microencapsulated naproxen suspension dosage form was the preferred one compared with the other two dosage forms as it was best in taste-masking of the drug naproxen, mouth feel, and aftertaste. The term "mouth feel" includes the presence or absence of various texture sensations, e.g., including slimy, gritty, burning, etc. The term "aftertaste" as used herein refers to a lingering objectionable taste sensation which persists after swallowing and can be characterized as a numbing, burning or bitter sensation.

## EXAMPLE 4

(Microencapsulation of ibuprofen with cellulose acetate phthalate (CAP))

Ibuprofen was microencapsulated with cellulose acetate phthalate using the same procedure and concentrations as set forth in Example 1 except that ibuprofen was used in place of naproxen. The ibuprofen individual microcapsule core payload was approximately 91 wt. % with the remainder being cellulose acetate phthalate microcapsule wall material.

## EXAMPLE 5

(Preparation of chewable tablets dosage form of ibuprofen micro-encapsulated with CAP)

Chewable tablets containing individual microcapsules having approximately 90.3 wt.% (Formulation A), and 90.6 wt. % (Formulations B and C) of the active ibuprofen microencapsulated with CAP were prepared (as in Example 1) using the same basic chewable tablet making procedure set forth above in Example 2, but using the below formulations, which differ depending upon the particular targeted patient population, as follows: Formulation A was prepared so that each chewable tablet had 50 mg of ibupro- fen. Formulation B was prepared to contain 100 mg of ibuprofen in chewable tablet form and Formulation C was prepared to contain 200 mg of ibuprofe/chewable tablet.

## Formulation A

(PROCEDURE FOR PREPARATION OF 2000 - 50 mg ACTIVE CHEWABLE IBUPROFEN TABLETS)

The following materials were added to a laboratory V-blender and blended for twenty minutes:

a. 110.7 grams of taste-masked ibuprofen micro-capsules -(90.3% active),

b. 400 grams of Mannitol USP granules,

c. 200 grams of Avicel PH-101,

d. 0.6 grams of FD&C Yellow #6,

e. 30 grams of Aspartame,

f. 6 grams of Citric Acid, and

g. 30 grams of Orange Flavor.

After blending the above materials for 20 minutes, a small portion of the blend was removed into a container and 2.6 grams of magnesium stearate was added. The materials were mixed by hand and the mixture was returned to the V-blender and blended for 10 minutes.

Tablets were compressed on a Stokes RB2 rotary press with 0.95 cm round, beveled edge, tooling.

The following resulting tablets had the following physical properties:

Hardness - 7.5 Strong-Cobb units

Friability - 0.47% (20 tablets at 25 rpm - 4 min.)

Average Weight - 395 milligrams

## Formulation B

(PROCEDURE FOR PREPARATION OF 1000 - 100 mg ACTIVE CHEWABLE IBUPROFEN TABLETS)

The following materials were added to a laboratory V-blender and blended for twenty minutes:

a. 110.5 grams of taste-masked ibuprofen micro-capsules -(90.6% active),

b. 300 grams of Mannitol USP granules,

c. 100 grams of Avicel PH-101,

d. 50 grams of Di Calcium Phosphate,

e. 15 grams of Aspartame,

f. 2 grams of Tartaric Acid, and

g. 10 grams of Peppermint Flavor.

After blending the above materials for 20 minutes, a small portion of the blend was removed into a container and 1.25 grams of magnesium stearate was added. The material were mixed by hand and the mixture was returned to the V-blender and blended for 10 minutes.

Tablets were compressed on Stokes RB2 rotary press with 3/8 inch round, beveled edge, tooling.

The resulting tablets had the following physical proper-ties:

Hardness - 10.3 Strong Cobb units

Friability - 0.38% (20 tablets at 25 rpm - 4 min.)

Average Weight - 595 milligrams

## Formulation C

(PROCEDURE FOR PREPARATION OF 1000 - 200 mg ACTIVE CHEWABLE IBUPROFEN TABLETS)

The following materials were added to a laboratory V-blender for twenty minutes:

a. 221 grams of taste-masked ibuprofen micro-capsules -(90.6% active),

b. 600 grams of Mannitol USP granules,

c. 200 grams of Avicel PH-101,

d. 100 grams of Di Calcium Phosphate,

e. 30 grams of Aspartame,

f. 4 grams of Tartaric Acid, and

g. 20 grams of Peppermint Flavor.

After blending the above materials for 20 minutes, a small portion of the blend was removed into a container and 2.5 grams of magnesium stearate was added. The materials were mixed by hand and the mixture was returned to the V-blender and blended for 10 minutes.

Tablets were compressed on a Stokes RB2 rotary press with 1.43 cm round, beveled edge, tooling.

The resulting tablets had the following physical properties:

Hardness - 13.5 Strong-Cobb units

Friability - 1.6% (20 tablets at 25 rpm - 4 min.)

Average Weight - 1178 milligrams

## EXAMPLE 6

(Preparation of a liquid suspension of CAP microencapsulated ibuprofen individual microcapsules having approximately 91 wt.% payload of ibuprofen)

Utilizing the procedure of Example 3 above, the CAP microencapsulated ibuprofen microcapsules (prepared as in Example 4) were formulated into liquid suspension dosage forms, depending upon the particular targeted patient population.

Formulation A was prepared using 2.2 grams of individual ibuprofen microencapsules per 100 mls of the liquid suspension vehicle prepared in Example 3 for a 100 mg per 5 ml active ibuprofen dose.

Formulation B was prepared using 4.4 grams of individual ibuprofen microcapsules per 100 mls of the Example 3 liquid suspension vehicle for a 200 mg per 5 ml active ibuprofen dosage.

Formulation C was prepared using 8.8 grams of individual ibuprofen microcapsules per 100 mls of the Example 3 liquid suspension vehicle for a 400 mg per 5 ml active ibuprofen dose.

### EXAMPLE 7

(Preparation of Cellulose Acetate Phthalate (CAP) Microencapsulated Ibuprofen)

Into a 3-liter beaker fitted with a birdcage baffle and a stirring motor with a 7.62cm turbine-blade, were added 30 grams of CAP into 600 ml. of deionized water.

With agitation, a sufficient quantity of sodium bicarbonate was added to adjust the pH so as to solubilize the CAP. The CAP has a particle size of about 40 mesh to 200 mesh. The CAP particles were well dispersed to facilitate dissolving the CAP polymer. The resulting solution contains 5 wt. % CAP and has a pH of about 7.0 or slightly above.

Next, 150 g of ibuprofen were dispered into the CAP solution. The ibuprofen particles had a particle size of approximately 25 to 500 $\mu$m 900 g of a 20% (wt/wt) aqueous sodium sulfate solution were added slowly (dropwise) with continuous agitation at about 150 rpm. This addition takes place over approximately 1-1/2 hours. This sodium sulfate aqueous solution acts as a coacervating agent permitting the CAP polymer to come out as a liquid phase microencapsulating material wrapping the ibuprofen drug particles.

After the CAP liquid microcapsule walls are formed, the CAP polymer was rendered insoluble by slow addition of 20% wt/wt citric acid solution until the final solution pH is approximately 4, thus rendering the CAP insoluble at that pH for water. Once this has been accomplished, the agitation is stopped and the microcapsules are allowed to settle from solution so that the supernatant liquid may be decanted so as to rid the batch of sodium sulfate.

These individual microcapsules of ibuprofen were then washed two times with 800 ml of water. The washed CAP microencapsulated ibuprofen microcapsules were then filtered by a vacuum filter and dried for three hours in an oven whose temperature is held at 45 deg. C. These individual microcapsules contain approximately 83-1/3 wt. %

### EXAMPLE 8

(Preparation of Cellulose Acetate Phthalate (CAP) Microencapsulated Ibuprofen)

Into a 3-liter beaker fitted with a birdcage baffle and a stirring motor with a 7.62 cm turbine blade, were added 30 grams of CAP into 600 ml. of deionized water.

With agitation, a sufficient quantity of sodium bicarbonate was added to adjust the pH so as to solubilize the CAP. The CAP has a particle size of about 40 mesh to 200 mesh. The CAP particles were well dispersed to facilitate dissolving the CAP polymer. The resulting solution contains 5 wt.% CAP and has a pH of about 7.0 or slightly above.

Next, 300 g of ibuprofen wars dispersed into the CAP solution. The ibuprofen particles bad a particle size of approx-imately 25 to 500 $\mu$m . 900 g of a 20% (wt/wt) aqueous sodium sulfate solution were added slowly (dropwise) with continuous agitation at about 350 rpm. This addition takes place over approximately 3 hours. This sodium sulfate aqueous solution acts as a coacervating agent permitting the CAP polymer to come out as a liquid phase microencapsulating material wrapping the ibuprofen drug particles.

After the CAP liquid microcapsule walls are formed, the CAP polymer was rendered insoluble by slow addition of 70 ml of a 20% wt/wt citric acid solution over a period of approximately 30 minutes until the final solution pH is approximately 4, thus rendering the CAP insoluble at that pH for water. Once this has been accomplished, the agitation is stopped and the microcapsules are allowed to settle from solution so that the supernatant liquid may be decanted so as to rid the batch of sodium sulfate.

These individual microcapsules of ibuprofen were then washed two times with 800 ml of water. The washed CAP micro-encapsulated ibuprofen microcapsules were then filtered and dried for 2-1/2 hours in an oven whose temperature is held at 45 deg. C. The semi-dry microcapsules were then passed through a 20 mesh sieve to break apart the agglomerates. The batch of microencapsulated ibuprofen is then placed in a tray and air dried at room temperature overnight. These individual microcapsules contain approximately 90.9 wt. % ibuprofen as payload of the active drug.

## EXAMPLE 9

(Preparation of Cellulose Acetate Phthalate (CAP) Microencapsu-lated Ibuprofen)

Into a 1-liter breaker fitted with a birdcage baffle and a stirring motor with a 7.62 cm turbine blade, were added 7.5 grams of CAP into 150 ml. of deionized water.

With agitation, a sufficient quantity of sodium bicarbonate was added to adjust the pH so as to solubilize the CAP. The CAP has a particle size of about 40 mesh to 200 mesh. The CAP particles were well dispersed to facilitate dissolving the CAP polymer. The resulting solution contains 5% (wt/wt) CAP and has a pH of about 7.0 or slightly above. This CAP solution was diluted with 150 ml of water.

Next, 75 g of ibuprofen (granulated with ethycellulose) were dispersed into the CAP solution. The ibuprofen granules had a particle size of approximately 150 to 500 $\mu$m. 225 ml of a 20% (wt/vol) sodium chloride buffer solution (USP 7.2 pH buffer was used) were added slowly (dropwise) with continuous agitation at about 150 rpm. This addition took place over approximately 1 hour. This aqueous sodium chloride buffer solution acted as a coacervating agent permitting the CAP polymer to come out as a liquid phase microencapsulating material wrapping the ibuprofen drug particles.

After the CAP liquid microcapsule walls were formed, the CAP polmer was rendered insoluble by slow addition of 15 ml of a 20% (wt/wt) citric acid aqueous solution over a period of approxi-mately 10 minutes until the final solution pH was approxi-mately 4, thus rendering the CAP insoluble at that pH for water. Once this had been accomplished, the agitation was stopped and the microcapsules were allowed to settle from solution so that the supernatant liquid could be decanted so as to rid the batch of sodium chloride buffer.

These individual microcapsules of ibuprofen were then washed two times with 200 ml of water. The washed CAP microencapsulated ibuprofen microcapsules were then filtered and dried for 2-1/2 hours in an oven whose temperature was held at 45 deg. C. The batch of microencapsulated ibuprofen was then placed in a tray and air dried at room temperature overnight. These individual microcapsules contained approximately 90.9 wt. % ibuprofen as payload of the active drug. The dry microcapsules were then passed through a 20-mesh sieve to break apart the agglomerates.

## EXAMPLE 10

(Preparation of Cellulose Acetate Phthalate (CAP) Microencapsu-lated Zomepirac, Sodium. (The commercial trade name for this drug is Zomax).

Into a 3-liter beaker fitted with a birdcage baffle and a stirring motor with a 7.62 cm turbine blade, were added 30 grams of CAP into 60 ml. of deionized water.

With agitation, a sufficient quantity of sodium bicarbonate was added to adjust the pH so as to solubilize the CAP. The CAP had a particle size of about 40 mesh to 200 mesh. The CAP particles were well dispersed to facilitate dissolving the CAP polymer. The resulting solution contained 5 wt. % CAP and had a pH of about 7.0 or slightly above. Next there was added 6 ml of a 1.3% (wt/vol) aqueous solution of dioctyl sodium sulfosuccinate material. The CAP solution was diluted with 600 ml of deionized water.

Next, 300 g of Zomax were dispersed into the CAP solution. The Zomax particles had a particle size of approximately 25 to 400 microns. 1000 ml of a 20% (wt/vol) aqueous sodium sulfate solution were added slowly (dropwise) with continuous agitation at about 150 rpm. This addition took place over approximately 2 hours. This sodium sulfate aqueous solution acted as a coacer-vating agent permitting the CAP polymer to come out as a liquid phase microencapsulating material wrapping the Zomax drug par-ticles.

After the CAP liquid microcapsule walls were formed, the CAP polymer was rendered insoluble by slow addition of a sufficient quantity of 20% (wt/wt) citric acid solution over a period of approxi-mately 10 minutes until the final solution pH was approxi-mately 4, thus rendering the CAP insoluble at that pH for water. Once this had been accomplished, the agitation was stopped and the micro-oapsules were allowed to settle from solution so that the supernatant liquid may be decanted so as to rid the batch of sodium sulfate.

These individual microcapsules of Zomax were then washed three times with 530 ml of a 1.75% (wt/vol) solution of aqueous citric acid. The washed CAP microencapsulated Zomax microcapsules were then filtered and dried for one hour in a fluid bed dryer whose temperature was held at 40 deg. C. The dry microcapsules were then passed through a 20-mesh sieve to break apart the agglomerates.

## Claims

1. A taste-masked, microencapsulated, non-steroidal, anti-inflammatory, water-insoluble drug material (NSAID) characterised in that it comprises individual microcapsules microencapsulated with cellulose acetate phthalate and having a high payload of active drug and characterized as free flowing individual, taste-masked microcapsules in the substantial absence of microcapsule agglomerates and having a microcapsule particle size distribution ranging from about 25 to about 600 μm.

2. A taste-masked microencapsulated water-insoluble NSAID drug material as claimed in Claim 1 wherein said drug is naproxen.

3. A taste-masked microencapsulated water-insoluble NSAID drug material as claimed in Claim 1 wherein said drug is ibuprofen.

4. A taste-masked microencapsulated water-insoluble NSAID drug material as claimed in anyone of Claims 1-3 wherein said payload of active drug is at least 83 + wt.%.

5. A taste-masked microencapsulated water insoluble NSAID drug material as claimed in anyone of Claims 1-3, wherein said payload of active drug is about 90 + wt.%.

6. A process for preparing microencapsulated non-steroidal, anti-inflammatory water-insoluble NSAID drug material which comprises preparing an aqueous dispersion of said NSAID material having a particle size distribution ranging from about 25 to about 500 μm within an aqueous solution of cellulose acetate phthalate at a pH of about 6 or higher and containing from about 2 weight percent to about 8 weight percent of cellulose acetate phthalate; gradually adding with continued agitation a solution containing an inorganic salt of the Hofmeister (lyotropic) series, to form cellulose acetate phthalate in liquid phase separate from the equilibrium liquid; slowly adjusting the pH of the resulting solution to a pH of about 4 or lower; while rendering said cellulose acetate phthalate polymer insoluble by slow addition of a dilute acid material; and recovering the individual microcapsules thus formed.

7. A process as claimed in Claim 6 wherein said water-insoluble NSAID drug material is naproxen.

8. A process as claimed in Claim 6 wherein said water-insoluble NSAID drug material is ibuprofen.

9. A process as claimed in anyone of Claims 6 - 8, wherein said Hofmeister (lyotropic) salt is sodium sulfate.

10. A process as claimed in anyone of Claims 6 - 9, wherein said dilute acid material is citric acid.

11. A taste masked, microencapsulated, water-insoluble, non-steroidal, anti-inflammatory drug (NSAID) in chewable tablet form containing microcapsules as claimed in anyone of Claims 1 - 5 and pharmaceutically acceptable excipient materials.

12. A liquid suspension comprising individual taste-masked microcapsules as claimed in anyone of Claims 1 - 5 and pharmaceutically acceptable excipient materials.

Claims for the following Contracting States: ES, GR

1. A process for preparing microencapsulated non-steroidal, anti-inflammatory water-insoluble drug (NSAID) material characterised in that the process comprises preparing an aqueous dispersion of said NSAID material having a particle size distribution ranging from about 25 to about 500 μm within an aqueous solution of cellulose acetate phthalate at a pH of about 6 or higher and containing from about 2 weight percent to about 8 weight percent of cellulose acetate phthalate; gradually adding with continued agitation a solution containing an inorganic salt of the Hofmeister (lyotropic) series, to form cellulose acetate phthalate in liquid phase separate from the equilibrium liquid; slowly adjusting the pH of the resulting solution to a pH of about 4 or lower; while rendering said cellulose acetate phthalate polymer insoluble by slow addition of a dilute acid material; and recovering the individual microcapsules thus formed.

2. A process as claimed in Claim 1 wherein said water-insoluble NSAID drug material is naproxen.

3. A process as claimed in Claim 1 wherein said water-insoluble NSAID drug material is ibuprofen.

4. A process as claimed in any one of Claims 1 - 3 wherein said Hofmeister (lyotropic) salt is sodium sulfate.

5. A process as claimed in anyone of Claims 1 - 4 wherein said dilute acid material is citric acid.

6. A process as claimed in any one of Claims 1 - 5, wherein the individual microcapsule payload of active water insoluble NSAID material is at least 83 weight %.

7. A process as claimed in Claim 6, wherein the payload of active drug is about 90 weight%.

8. A process for preparing a chewable tablet containing a microencapsulated water-insoluble NSAID, characterised in that a micro-encapsulated water-insoluble NSAID is prepared by a process as claimed in any one of Claims 1 - 7, and then mixed with pharmaceutical excipients and formed into chewable tablets.

9. A process for preparing a liquid suspension of a micro-encapsulated NSAID characterised in that a microencapsulated water-insoluble NSAID is prepared by a process as claimed in any one of Claims 1 - 7, and then mixed with pharmaceutical excipients to form a liquid suspension.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | Journal of Pharmaceutical Sciences vol. 62, no. 9, 1973, England pages 1444 - 1448; MERKLE, H.P. et al.: "Preparation and in vitro evaluation of cellulose acetate phthalate coacervate microcapsules" * page 1445, column 1, line 1 - page 1445, column 1, line 27 * | 1-12 | A 61 K 9/50 A 61 K 9/52 |

----

|  |  |  | TECHNICAL FIELDS SEARCHED (Int. Cl.5) |
|---|---|---|---|
|  |  |  | A 61 K |

The present search report has been drawn up for all claims

| Place of search | Date of completion of search | Examiner |
|---|---|---|
| The Hague | 28 November 90 | LEHERTE C.F.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document